# EUROPEAN PATENT APPLICATION

(11) **EP 4 205 644 A1**
(43) Date of publication of application: **05.07.2023**
(21) Application number: 21383240.5
(22) Date of filing: 30.12.2021
(51) Int. Cl.: A61B 5/024, A61B 5/145, A61B 5/00

(54) **A WEARABLE DEVICE FOR CONTINUOUS MONITORING OF HEALTH PARAMETERS**

(71) Applicant: Onalabs Inno-Hub, 08012 Barcelona (ES)
(72) Inventor: RABOST GARCIA, Genís, 08012 Barcelona (ES); MUÑOZ PASCUAL, Francesc Xavier, 08012 Barcelona (ES); AGUILAR TORÁN, Javier, 08012 Barcelona (ES); PUNTER VILLAGRASA, Jaime, 08012 Barcelona (ES); COLMENA RUBIAL, Valeria, 08012 Barcelona (ES)
(74) Representative: Hoffmann Eitle

(57) **Abstract**

The present invention refers to a device for the continuous monitoring of health condition of patients or sport persons without the need of blood extraction. In particular the invention refers to a wearable device that comprises: at least one sweat sensor for measuring a sweat biomarker, a sweat collection inlet arranged in the device for collecting sweat when the device is worn by a user, a microfluidic channel for conveying collected sweat from the inlet to the sweat sensor, at least one vital-sign sensor arranged in the device for measuring a vital-sign or physiological sign of the user, a sweat volume sensor for measuring volume of the collected sweat, and a processing unit adapted for receiving and processing data provided by the sweat sensor, the vital-sign biosensor and the sweat volume measuring device. The invention can advantageously be used in sports medicine and/or sports health sectors for remote effort and/or fatigue assessment.

## Description

### Field and object of the invention

The present invention belongs to the technical filed of medical devices, in particular to the area of wearable medical devices both for clinical and sport applications.

An object of the invention, is the provision of a multi-sensor device for the continuous remote, and real-time monitoring of health condition of patients or sport persons, without blood extraction.

The invention can advantageously be used in sports medicine and/or sports health sectors for remote effort and/or fatigue assessment.

### Background of the invention

Blood tests are the reference clinical methods that show the most important physiological parameters that allow an accurate diagnosis of a patient's condition. In the health sector, the analysis of most of the metabolites of interest (various amino acids, sugars, carboxylic acids and fatty acids, ...) requires blood collection, creating an obstacle for their use in real-time monitoring in both clinical health and sports medicine.

Therefore, there is a need in the medical personnel in charge of clinical protocols as well as in the training and recovery of the athletes, for devices capable of monitoring biomarkers in a non-invasive, continuous and remote way by means of sweat. In this regard, although the use of blood is the reference, analyzing sweat has been a challenge to obtain bioequivalence between both measurement formats.

The current clinical trend is to be less invasive contemplates the tendency to combine both non-invasive measurement techniques: on skin and on biofluid (urine, saliva, sweat, etc.).

To measure sweating volume and rate, there are different strategies like: using humidity sensors, calorimetric measurements or volumetric determination. Using humidity sensors or calorimetric sensors are highly sensitive, but require a high level of complexity that limits their integration into portable devices. In contrast, volumetric measurements, determining the volume collected in a controlled period of time, allows simple and direct monitoring. There are visual monitoring options that are simple for the research setting as they require little instrumentation. However, for continuous monitoring applications it is better to use impedimetric detection (electrical resistance is reduced proportionally to the sample volume) which allows autonomous and real-time measurement.

### Summary of the invention

The present invention is defined in the attached independent claim, and satisfactorily solves the drawbacks of the prior art, by providing a wearable device that integrates different sensors to obtain simultaneous measurements of biomarkers in sweat (metabolites, ions or amino acids, etc.), and vital physiological parameters (heart and respiratory rate, blood pressure, etc.), for the determination of lactate concentration in blood, in a non-invasive, continuous, real-time and remote manner.

More specifically, an aspect of the invention refers to a wearable device for continuous monitoring of health parameters of a user, wherein the device comprises: at least one sweat sensor for measuring a sweat biomarker, a sweat collection inlet arranged in the device for collecting sweat when the device is worn by a user, and a microfluidic channel for conveying collected sweat from the inlet to the sweat sensor.

Additionally, the device comprises: at least one vital-sign sensor arranged in the device for measuring a vital-sign or physiological sign of the user, a sweat volume sensor for measuring volume of the collected sweat, and a processing unit adapted for receiving and processing data provided by the sweat sensor, the vital-sign biosensor and the sweat volume measuring device.

Preferably, the vital-signs or signs sensors is one or more of the following sensors: a heart rate sensor, a respiratory rate sensor, a blood pressure sensor, a body temperature sensor, and an oxygen saturation sensor.

The processing unit is further adapted to calculate or predict a blood lactate concentration based on data provided by the sweat sensor, the sweat volume sensor, and a vital sign-sensor. Preferably, the data is the form of electric signals that can be processed by the processing unit to perform calculations based on that data.

In a preferred embodiment, the sweat sensor is a sweat lactate sensor and the vital-sign sensor is a heart rate sensor, and the processing unit is further adapted to calculate, preferably by means of machine learning algorithms in a known manner for a skilled person in the art, predictive values of lactate concentration in blood, based on data in the form of electric signals, provided by: the sweat lactate sensor, the sweat volume sensor and heart rate sensor.

Therefore, the wearable device of the invention is a smart wearable device for lactate monitoring, that integrates a microfluidic system that ensures efficient sweat capture and continuous circulation of a fresh sweat sample. The invention is capable of providing real time health status of athletes and report when an athlete enters the anaerobic phase, thus, creating a new paradigm in sports training planning, as well as the health status of the athlete.

A direct and proportional relationship between lactate measurement (key biomarker in the monitoring of intensive physical exertion or fatigue) in blood and sweat is unknown, thus, in the present invention it has been found that taking into account additional parameters to sweat lactate, would establish a more accurate bioequivalence with blood lactate.

In particular, the invention involves the processing of the following additional parameters to sweat lactate for the blood lactate estimation: sweat rate, sweat volume excreted by the subject, and heart rate. In the invention, the sweating rate is also taken into account as a factor of dilution of lactate and the other analytes present in sweat.

The sweating rate gives an indication of the decrease in sweat lactate concentration in the tests and the variation observed with the blood lactate trend. Moreover, the heart rate provides a good estimation of the intensity of the physical activity performed by a user, and it is a common parameter in the monitoring of the athlete's condition and fatigue.

The wearable device of the invention integrates a microfluidic system for collecting sweat, that in contrast to prior art devices that are only capable of providing time isolated sweat measurements of no practical use, includes a sweat sample renewal system, such that fresh sweat measurement is always performed, which provide a faithful indication of the physical effort made at that time by a user.

Furthermore, the device incorporates a communication module adapted for the wireless transmission of data processed by the processing unit, preferably for transmitting the estimated or calculated blood lactate concentration.

The device is provided with a sensing chamber and at least one of the following sensors placed in the sensing chamber: a sweat lactate sensor, a sweat conductivity sensor, metabolites sensor, ions sensors, amino acids sensor. The microfluidic channel communicates the sweat inlet with the sensing chamber.

The sweat volume sensor comprises a microfluidic circuit or a microfluidic reservoir, fluidly communicated with the sensing chamber and arranged downstream the sensing chamber, and a pair of electrodes such that the microfluidic reservoir is arranged in between the two electrodes, in a way that a capacitance value between the two electrodes is variable depending on the amount of sweat in the reservoir. The two electrodes are a pair of strips opposite each other, and more preferably the electrodes are embodied as conductive flexible strips.

The microfluidic reservoir can have any form, for example the microfluidic reservoir can be a straight or curved conduit, or it can be formed as a conduit having a meander configuration.

Preferably, the wearable device comprises three main parts, namely: a main housing, means for attaching the main housing to a part of the user's body, and a fungible or consumable part disposable after use, which is configured to be manually attachable and detachable from the main housing.

The means for attaching the main housing to a part of the user's body, preferably consist of a flexible band having two ends respectively couplable with the main housing.

In a preferred embodiment, the processing unit is integrally contained in the main housing, but in other preferred embodiments of the invention, the processing unit or part of it, is external to the device, for example, it is implemented in a smart phone, a smart watch, a tablet or similar devices. The main housing additionally includes a power source like a battery, for supplying power to the electronics implementing the processing unit, and electric connectors for the communication with the consumable part.

The flexible band is fitted with at least one biosensor for measuring a vital-sign or physiological sign of the user, such as a heart rate sensor, thus, in addition to the sweat measurements, heart rate measurements are also taken in direct contact with the skin, with the same de device, and simultaneously as the sweat parameters are measured.

The flexible band is implemented as an elastic textile tape that would have a good hold on the body and would contain a pair of electrodes to measure the heart rate. The electrodes are made of bioelectric silicone to capture the electrocardiogram signal and extract the heart rate. They have sufficient conductivity to obtain a quality heart rate signal and are resistant to continuous use and washing.

The flexible band can be provided with other sensors, like a respiratory rate sensor to obtain additional parameters.

The consumable component has a contact surface provided to be in contact with the user's skin when the main housing is attached to a user's body part, and the consumable component is coupled with the main body.

Alternatively, the means for attaching the main housing to a part of a user's body, comprises an adhesive material for example an adhesive surface suitable to be adhered on a user's skin. For example, this adhesive surface is provided on a surface of the consumable component meant to be in contact with the user's skin.

The consumable component incorporates: the sweat collection inlet formed in the consumable component's contact surface at least one sweat sensor, the sweat rate measuring device, and the microfluidic channel, and electric connection means for electrically connecting the consumable component with the processing unit when the consumable component is coupled with the main housing.

Preferably, the consumable component is a generally flat body embodied as a card-like component.

In a preferred implementation of the wearable device, the main housing has a front surface and a back surface provided with a pair of guides opposite each other, and the consumable component has a pair of sides wings, such that the consumable component is couplable with the main housing by inserting its side wings respectively in the guides and by moving the consumable component on the back surface of the main housing.

A part of the back surface of the main housing is generally flat, and the consumable component is couplable with the main housing by moving the consumable component on a plane parallel to said generally flat part of the back surface.

The main body is provided by a pair of electric connectors and each end of the flexible band is fitted with metallic connectors for mechanically and electrically connecting the flexible band and the biosensor with the main body.

The main body includes a battery for supplying power to the processing unit, and the main body has a lid at its back surface for providing access to the battery. The consumable component is overlapped with the lid, when the consumable component is operatively coupled with the main housing.

The wearable device of the invention, provides an advanced and integral personalized health management capability, through the combined analysis of key metabolic and physiological health indicators, like blood glucose measurement data, in addition to heart rate, oxygen saturation and temperature measurements, to achieve accurate equivalence with blood glucose measurements.

In summary, some of the main advantages of the invention are the followings:
- the diagnostic capacity of a biofluid like sweat is increased; much more patient data is generated by monitoring in a non-invasive, continuous and remote way;
- the format of the wearable device, is already used by athletes (heart rate monitor) to which they are accustomed in their regular training;
- the permanent part of the device (main housing) can be used by itself, in case the chemical measurement with the fungible or consumable part is not necessary (already known training routine, etc...);
- the permanent part (main housing) is compatible with different consumable part depending on the parameters to be measured. Useful tool not only for the end user but also for the clinic/sports physician who can adapt the device to the requirements of the patient/study;
- simple to use by insertion of the consumable and placement of the heart rate monitor. Guarantees placement in the same place every time, limiting the variability that the user's own operation may entail;
- it allows to have more advanced electronics with advanced functionalities compared to patch wearables such as battery management, data processing, flash memory in case there is no possibility of communicating the data;
- the consumable part might have an adhesive surface to enhance its fastening with the permanent part.

The main application of the invention is focused on sports medicine or sports health sector, for example for monitoring dehydration monitoring in athletes, but depending on the combination of sensors used, the device can be adapted to different applications as explained below.

Therefore, in a preferred embodiment, the wearable device is adapted to operate as a device for dehydration monitoring in athletes. A combination of sensors for measuring sweating, conductivity and some salt such as sodium can give real-time information on the state of dehydration of an athlete during physical exertion. Important to improve the control of dehydration in tests, and to be able to be treated in time (as an alarm system for the user) and with measurable variables in order to create an objective scale.

In another preferred embodiment, the wearable device comprises a heart rate and sweat rate measurement sensors, and the device is adapted to operate as a device for monitoring user fatigue.

In another preferred embodiment, the wearable device is adapted to operate as a device for nocturnal hypoglycaemia monitoring. In this embodiment, the device is a non-invasive device provided with sensors for measuring: heart rate, sweating, conductivity and a glucose sensor adapted to low ranges. The user would put the device on during the night, and the device can generate alarms when an episode of this type is detected. With the addition of predictive models, the onset of an episode could be predicted in advance to prevent and limit its consequences.

In another preferred embodiment, the wearable device is adapted to operate as a device for peritoneal dialysis remote monitoring. In this embodiment, the device comprises a heart rate sensor, an accelerometer, a sweat rate sensor, and sensors for measuring: conductivity, ions such as sodium, and lactate.

### Brief description of the drawings

Preferred embodiments of the invention are henceforth described with reference to the accompanying drawings, wherein:
Figure 1.- shows a perspective view of a preferred embodiment of the invention including an attachment band or strap.
Figure 2.- shows a perspective view of the main housing and the consumable part partially coupled together.

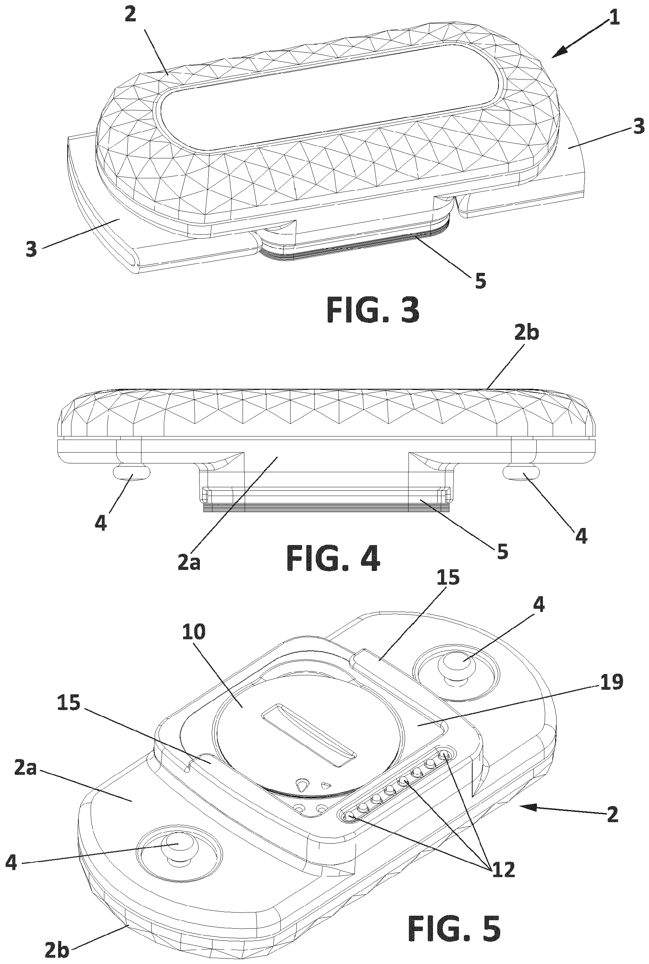

Figure 3.- shows another perspective view of the main housing from above, and part of the band ends.
Figure 4.- shows a front elevational view of the main housing.
Figure 5.- shows a perspective view of the main housing from below.
Figure 6.- shows an exploded view of the main housing components.
Figure 7.- shows in Figure A a perspective view of the main housing from below. In Figure B with the consumable part partially coupled, and in Figure C with the consumable part fully coupled.
Figure 8.- shows an schematic representation of the sweat volume sensor. The figure represents four stages (A - B) of filling of the microfluidic channel.

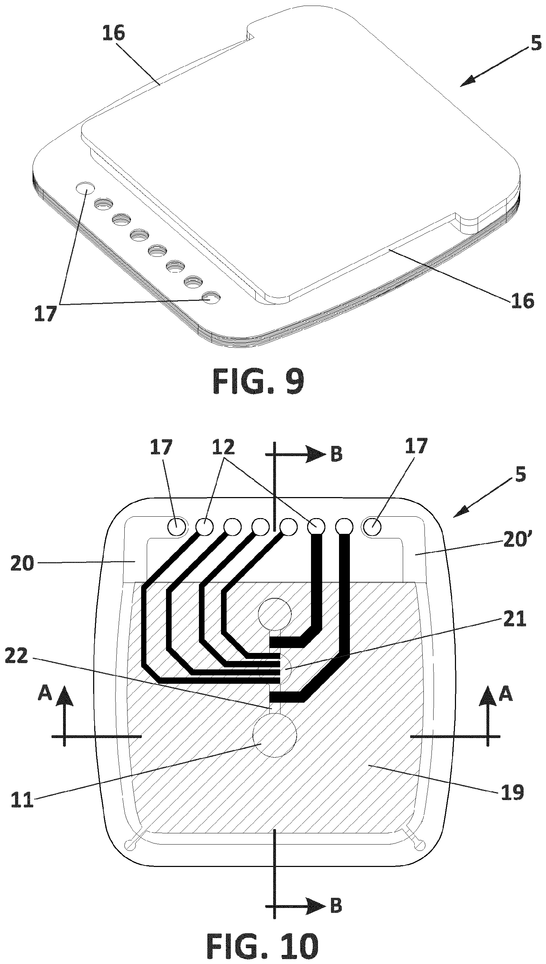

Figure 9.- shows a perspective view of the consumable device from the face opposite the surface meant to be in contact with the skin.
Figure 10.- shows a schematic representation in plan view of the consumable device.
Figure 11.- shows a cross-sectional view of the consumable device, taken at cutting plane A-A in figure 10.
Figure 12.- shows another cross-sectional view of the consumable device, taken at cutting plane B-B in figure 10.
Figure 13.- shows a graph illustrating the characterization of the sweat volume sensor.
Figure 14.- shows a graph illustrating the effects of sweat conductivity on measured capacitance of the sweat volume sensor.

### Preferred embodiment of the invention

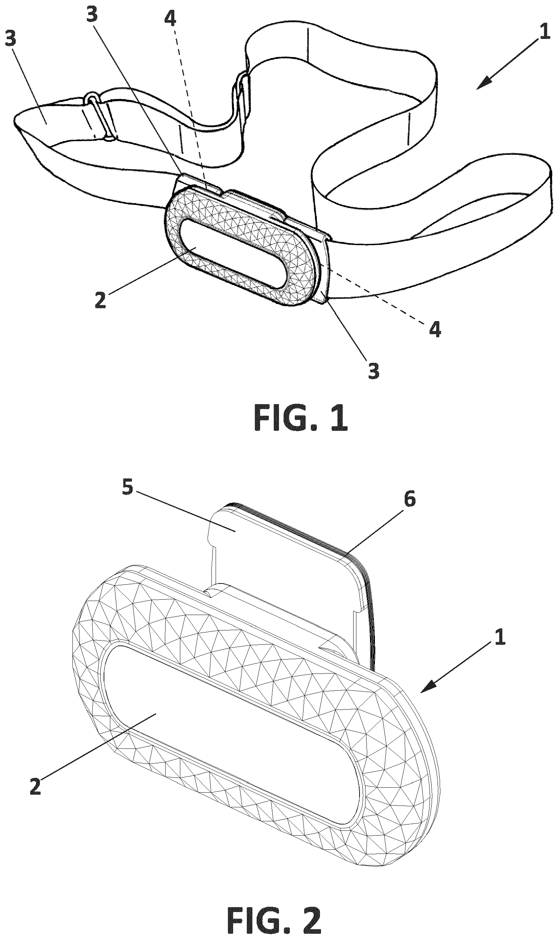

Figure 1 shows an exemplary embodiment of a wearable device (1) according to the invention comprising a main housing (2) and a flexible band or strap (3) for attaching the main housing to a part of a user's body, and a pair of electric and mechanic connectors or snap buttons (4) provided in the main housing (2) for mechanically and electrically connecting the flexible band ends with the main housing (2).

The flexible band (3) is fitted conventionally with at least one biosensor for measuring a vital-sign or physiological sign of the user, in a known manner. The vital-signs or signs sensors are at least one of the following: a heart rate sensor, a respiratory rate sensor, blood pressure sensor, body temperature sensor, and oxygen saturation sensor.

As shown more clearly in **Figure 2****,** the device (1) includes a consumable component (5) configured to be manually coupled and uncoupled with the main housing (2), and having a contact surface (6) provided to be in contact with the user's skin when the consumable component (5) is coupled with the main housing (2), and the main housing (2) is attached to a user's body.

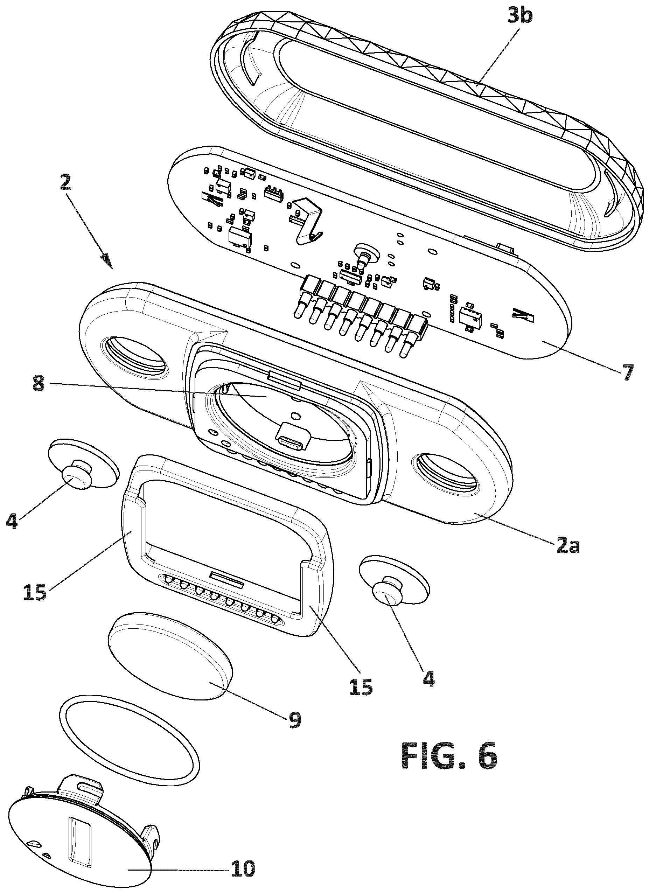

As shown in **Figure 6****,** the main housing (2) is formed by two couplable parts, a base (2a) and a lid or cover (2b) that configure a space within which an electronic circuit (7) is enclosed. This electronic circuit (7) implements the processing unit, sensors instrumentation, battery management, data processing and communication module for the wireless transmission of data processed by the processing unit.

The main housing (2) in particular the base (2a) has a receptacle (8) for receiving a battery (9) for supplying power to the electronic circuit (7), and a lid (10) for closing and opening the receptable (8).

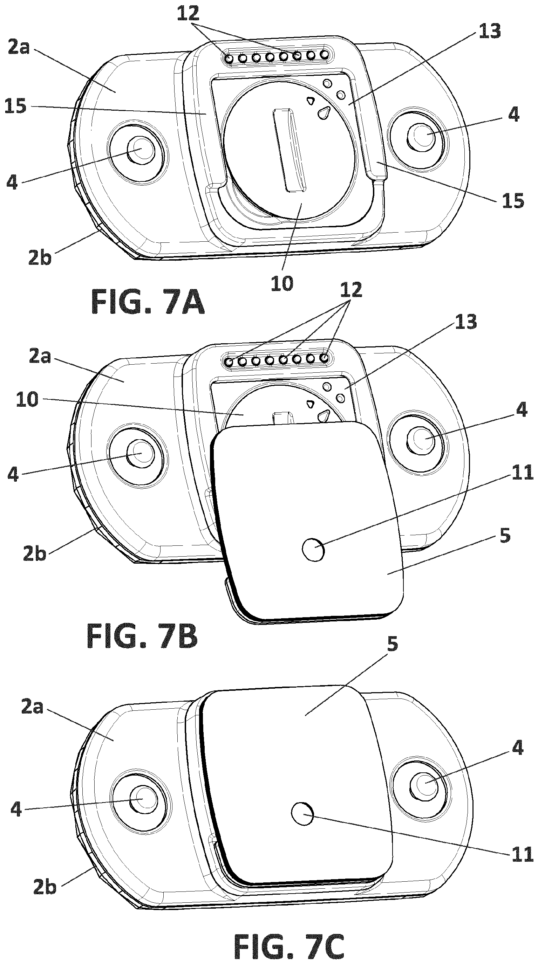

The consumable component (5) is generally a flat body, and the main housing (2) and the consumable component (5) are configured, such that the consumable component (5) is couplable with the main housing (2) by moving the consumable component (5) on the same lane as the generally flat back surface (19) of the main housing (2) or a plane parallel to it, as illustrated in **Figures 2****,** **7B** and **7C****,** such that the lid (10) can only be accessed when the consumable component (5) has been taken off from the main housing (2).

In particular, the consumable component (5) is overlapped with the lid (10) when the consumable component (5) is operatively coupled with the main housing (2), as it can be observed in **Figures 7A - 7C****.**

The consumable component (5) incorporates: a sweat collection inlet (11) formed in the consumable component's contact surface (6) for collecting sweat when the device is worn by a user, at least one sweat sensor, a sweat volume measuring device, and a microfluidic channel for conveying collected sweat from the inlet (11) to the sweat sensor.

In particular, consumable component (5) has a sensing chamber and at least one of the following sensors: a sweat lactate sensor, a sweat conductivity sensor, metabolites sensor, ions sensors, amino acids sensor, and, placed in the sensing chamber. The microfluidic channel communicates the sweat inlet (11) with the sensing chamber.

The consumable component (5) is manufactured as a stack of layers of plastic materials where the microfluidic channels are formed by laser cutting or die cutting. The integrated sensors are electrochemical in nature and therefore require electrodes that are fabricated by screen printing.

A set of electric connectors (12) are provided at the back surface (13) of the base (2a) of the main housing (2), in order to realise electric communication between the sensors located in the consumable component (5) and the processing unit (7). These connectors (12) are known spring-biased connectors, that stablish electric contact with corresponding electric pads (17) (provided in the consumable component (5), in a known manner when this is coupled with the main housing (2).

The consumable component (5) can be mechanically coupled and uncoupled from the main housing (2), in a quick and intuitive manner for a user, while ensuring functionality and good electrical connection with the main housing.

The main housing (2) has a back surface (13) provided with a pair of guides (15) opposite each other, and the consumable component (5) has a pair of sides wings (16), such that the consumable component (5) is couplable with the main housing (2) by inserting its side wings (16) respectively in the guides (15), and by moving the consumable component on the back surface (13) of the main housing (2).

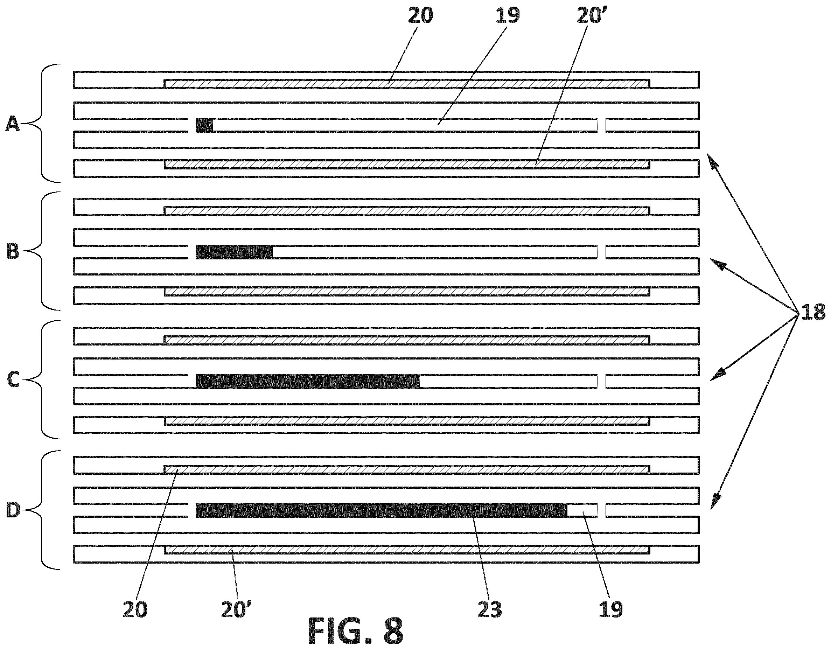

The sweat volume sensor (18) is represented schematically in **Figure 8****,** and comprises a microfluidic circuit or a microfluidic reservoir (19), fluidly communicated with the sensing chamber and arranged downstream the sensing chamber. In addition, the sweat volume sensor (18) comprises a pair of electrodes (20,20') and the microfluidic reservoir (19) arranged in between the two electrodes, such that a capacitance value between the two electrodes is variable depending on the amount of sweat in the reservoir.

The entry of sweat into the microfluidic reservoir (19) results in a change in capacitance between the two electrodes (dielectric constant difference between air and water which is the main component of sweat). This approach, apart from giving a very robust response with the added volume, has integration advantages, as it is only necessary to add two layers of copper tape in the lamination of the device, which is a cheap and easily available material. In addition, it does not require the fabrication of electrodes inside the channel and has sensitivity over the entire measurement area of the copper layers.

The electrodes (20,20') allow determination of the flow of sweat as it advances filling the reservoir (19) with limited influence of the ionic strength of the sweat sample.. The local sweat flow for each measurement interval can be estimated (we know the time and volume of sweat advancement) and thus be able to correct the sweat lactate measurement as a dilution factor.

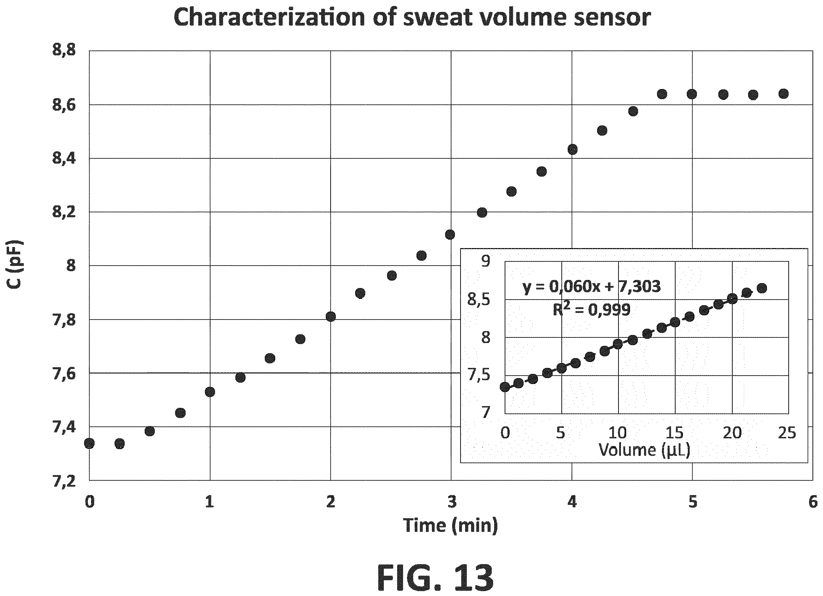

The experimental validation of this novel sweat volume sensor has been carried out by injecting a controlled volume with a syringe pump into the microfluidic reservoir while monitoring the capacitance between the two electrodes. The microfluidic reservoir consisted in a long serpentine microfluidic channel, fabricated using adhesive materials and laser cutting, entrapped between the electrodes made of copper conductive tape. A 100 mM NaCl solution, which replicates the typical ionic strength of sweat, was injected at a constant flow rate of 5 µL/min. In **Figure 13****,** it can be seen that, as the injection starts, the capacitance starts increasing linearly until the solution reaches the end of the sensing region. Then, the capacitance will remain stable although we keep injecting fluid because the sensing region is already completely filled. Converting time to volume injected, the calibration curve between capacitance and volume for the sweat volume sensor can be build.

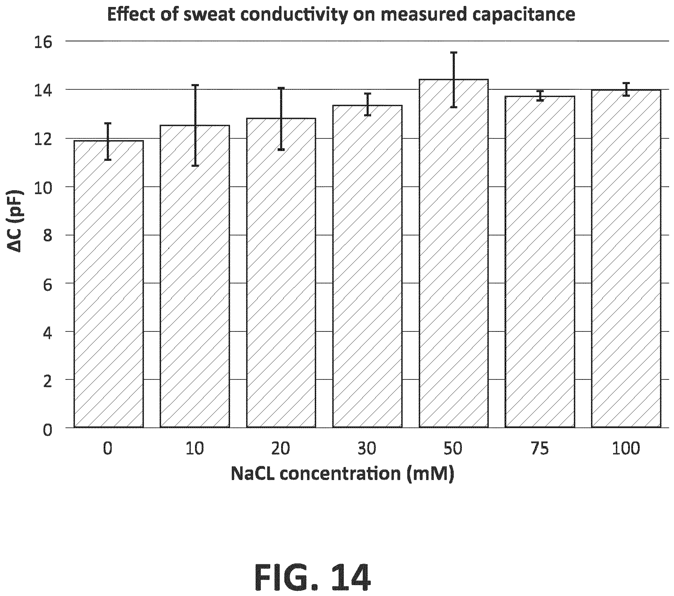

Most impedimetric sweat volume or rate sensors have a response which depends significantly on the conductivity or ionic strength of the sweat sample. This is because they have a resistive nature, where free ions present in sweat have a greater effect than in capacitive measurements, as the sensor proposed here. This can be observed in **Figure 14** where the increment in capacitance (between an empty and filled sweat volume sensor) is measured for different solutions of increasing ionic strength (NaCI solutions ranging from 0 to 100 mM). There is a slight increase in the increment of capacitance when the solution contains a higher concentration of ions, but for the range of interest (low, 30 mM, to high, 100 mM, concentrations) the change produced by the conductivity of the sample is found between the margins of the variability of the sensor.

Preferably, the two electrodes are a pair of conductive flexible strips opposite each other.

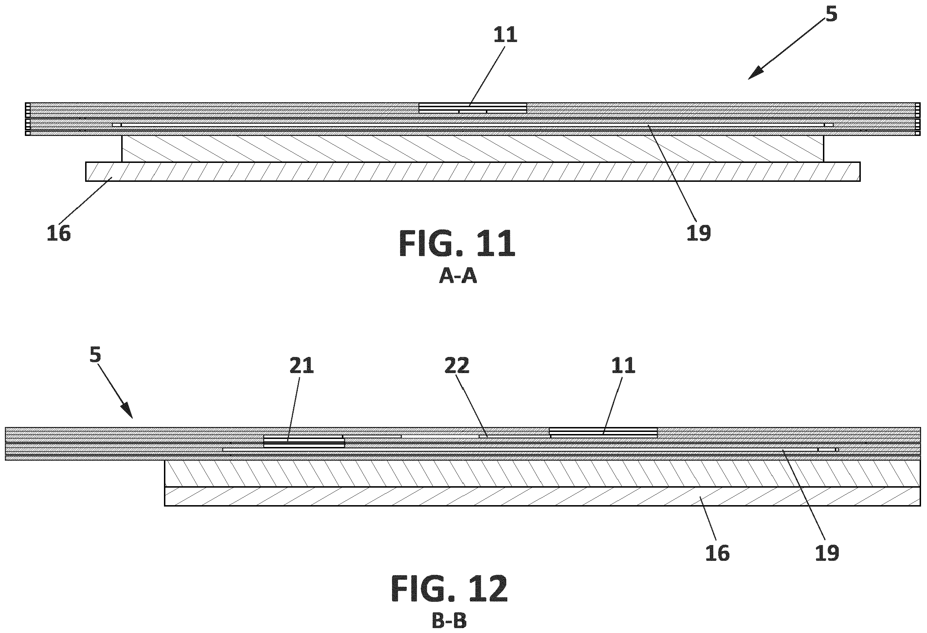

As shown in **Figures 11** and **12****,** in this particular embodiment, the sweat inlet (11), the microfluidic channel (22) and the sensing chamber (21), are placed above the microfluidic reservoir (19).

## Claims

1. A wearable device for continuous monitoring of health parameters of a user,
the device comprising:
at least one sweat sensor for measuring a sweat biomarker,
a sweat collection inlet arranged in the device for collecting sweat when the device is worn by a user,
a microfluidic channel for conveying collected sweat from the inlet to the sweat sensor,
at least one vital-sign sensor arranged in the device for measuring a vital-sign or physiological sign of the user,
a sweat volume sensor for measuring volume of the collected sweat, and
a processing unit adapted for receiving and processing data provided by the sweat sensor, the vital-sign biosensor and the sweat volume measuring device.

2. A wearable device according to claim 1, wherein the sweat sensor is a sweat lactate sensor, and the vital-sign sensor is a heart rate sensor, and wherein the processing unit is further adapted to calculate, preferably by means of machine learning algorithms, a blood lactate concentration based on data provided by: the sweat lactate sensor, the sweat volume sensor and heart rate sensor.

3. A wearable device according to claim 1 or 2, further comprising a communication module adapted for the wireless transmission of data processed by the processing unit.

4. A wearable device according to any of the preceding claims further comprising sensing chamber and at least one of the following sensors: a sweat lactate sensor, a sweat conductivity sensor, metabolites sensor, ions sensors, amino acids sensor, and, placed in the sensing chamber, and wherein the microfluidic channel communicates the sweat inlet with the sensing chamber.

5. A wearable device according to any of the preceding claims, comprising at least one of the following vital-signs or signs sensors: a heart rate sensor, a respiratory rate sensor, blood pressure sensor, body temperature sensor, and oxygen saturation sensor.

6. A wearable device according to any of the preceding claims, wherein the sweat volume sensor comprises a microfluidic circuit or a microfluidic reservoir, fluidly communicated with the sensing chamber and arranged downstream the sensing chamber.

7. A wearable device according to claim 6, wherein the sweat volume sensor comprises a pair of electrodes and a microfluidic reservoir fluidly communicated with the sensing chamber and arranged relative to each other, such that a capacitance value between the two electrodes is variable depending on the amount of sweat in the reservoir.

8. A wearable device according to claim 7, wherein the two electrodes are a pair of strips opposite each other, and the microfluidic reservoir is arranged in between the two electrodes, wherein preferably the electrodes are embodied as conductive strips, and more preferably the electrodes are embodied as conductive flexible strips.

9. A wearable device according to any of the preceding claims, further comprising:
a main housing having at least part of the processing unit enclosed therein,
means for attaching the main housing to a part of the user's body,
a consumable component configured to be manually attachable and detachable from the main housing, and having a contact surface provided to be in contact with the user's skin when the main housing is attached to a user's body part, and the consumable component is coupled with the main housing,
wherein the consumable component incorporates: the sweat collection inlet formed in the consumable component's contact surface at least one sweat sensor, the sweat rate measuring device, and the microfluidic channel, and
electric connection means for electrically connecting the consumable component with the processing unit when the consumable component is coupled with the main housing.

10. A wearable device according to claim 9, wherein the means for attaching the main housing to a part of a user's body, comprises a flexible band having two ends respectively couplable with the main housing, and wherein the flexible band is fitted with at least one biosensor for measuring a vital-sign or physiological sign of the user.

11. A wearable device according to claim 9, wherein the means for attaching the main housing to a part of a user's body, comprises an adhesive surface suitable to be adhered on a user's skin, and wherein preferably the adhesive surface is provided on the consumable component's contact surface.

12. A wearable device according to any of the claims, comprising a heart rate sensor and a sweat rate sensor, and wherein the device is adapted to operate as device for monitoring user fatigue.

13. A wearable device according to any of the claims 1 to 11, comprising a sweating sensor, a conductivity sensor and a ion sensor, and wherein the device is adapted to operate as a device for dehydration monitoring in athletes, based on data provided by the sweating sensor, the conductivity sensor and the ion sensor.

14. A wearable device according to any of the claims 1 to 11, comprising: a heart rate sensor, a sweating sensor, a conductivity sensor, and a glucose sensor preferably adapted to detect glucose concentrations below 55 mg/L, and wherein the device is adapted to operate as device for nocturnal hypoglycaemia monitoring based on data provided by the heart rate sensor, the sweating sensor, the conductivity sensor and the glucose sensor.

15. A wearable device according to any of the claims 1 to 11, comprising a heart rate sensor, an accelerometer, a sweat rate sensor, a conductivity sensor, an ions sensor preferably a sodium sensor, and a lactate sensor, and wherein the device is adapted to operate as a device for peritoneal dialysis monitoring based on data provided by the heart rate sensor, the accelerometer, the sweat rate sensor, the conductivity sensor, the ions sensor and the lactate sensor.
